# EUROPEAN PATENT APPLICATION

(11) **EP 4 817 542 A1**
(43) Date of publication of application: **07.10.2026**
(21) Application number: 25167827.2
(22) Date of filing: 01.04.2025
(51) Int. Cl.: A61B 18/14, A61B 18/00

(54) **A CATHETER-BASED SYSTEM AND METHOD OF USE**

(71) Applicant: AF-ABLATION S.R.L., 20121 Milan (MI) (IT)
(72) Inventor: PAPPONE, Carlo, 23870 Lecco (IT)
(74) Representative: Bird & Bird Società tra Avvocati S.r.l.

(57) **Abstract**

The innovative catheter-based system comprises a catheter (1000) able to map, ablate, and/or to deliver at least a therapeutic agent into at least a target area of a cardiac tissue, the catheter (1000) includes a therapeutic ablator device (100) disposed in a distal portion of the catheter (1000), the therapeutic ablator device (100) comprises: a drug delivery portal (110), able to drug locally released; a plurality of railings (120) arranged symmetrically and parallel to the drug delivery portal (110). The innovative method (2000) of use of the catheter-based system.

## Description

### TECHNICAL FIELD

The subject-matter disclosed herein relates to a catheter- based system in particular a catheter-based therapy delivery system, preferably in the treatment of arrhythmias and cardiac autoimmunity.

### BACKGROUND ART

Cardiac arrhythmias are a leading cause of morbidity and mortality worldwide, with sudden cardiac death (SCD) representing a significant consequence of high-risk electrophysiological disturbances. Traditionally, these arrhythmias have been attributed to genetic mutations affecting ion channel function, particularly in voltage-gated sodium (NaV1.5), potassium (hERG/KCNH2), and calcium (CaV1.2) channels.

However, from a scientific article with the title "Relationship among complex signals, short cycle length activity, and dominant frequency in patients with long-lasting persistent AF: a high-density epicardial mapping study in humans" (Geoffrey Lee et al., 2011), is demonstrated that a substantial subset of these disorders arises from autoimmune-mediated mechanisms, in which circulating autoantibodies target critical ion channels and regulatory proteins in the myocardium, leading to electrical instability and arrhythmogenesis.

As a result, a new category of disorders driven by immune dysregulation rather than primary genetic or structural abnormalities: "Lethal Autoimmune Channelopathies". Unlike inherited channelopathies, these conditions are marked by an adaptive immune response directed against cardiac ion channels, leading to functional dysregulation of key electrophysiological pathways. While autoimmune diseases such as lupus and rheumatoid arthritis have long been associated with cardiovascular complications, recent discoveries have highlighted that autoimmune cardiac arrhythmias can also develop independently of systemic autoimmune disorders. In these cases, pathogenic autoantibodies selectively interfere with ion channel function, ultimately predisposing individuals to life-threatening arrhythmias, as can be seen in the scientific article titled "Cardiac Arrhythmias in Autoimmune Diseases" (Monika Gawalko et al., 2020).

Recent evidence suggests that autoimmune mechanisms play a significant role in the development of cardiac arrhythmias, expanding the concept of "lethal autoimmune channelopathies" beyond inherited disorders. These findings highlight the need for targeted immunomodulatory approaches, such as the catheter-based system proposed in this patent." (Lazzerini PE et al., 2025)

In particular, the molecular mechanisms underlying autoimmune-driven arrhythmias involve a complex interplay of autoantibody production, inflammatory cytokines, and cellular immune responses that directly modulate the electrophysiological landscape of the heart. Autoantibodies targeting cardiac ion channels can contribute to arrhythmias through two primary mechanisms:
- direct functional inhibition or activation, wherein the autoantibodies can bind to ion channels and either block, potentiate, or alter their gating kinetics, leading to abnormal conduction properties. For example, NaV1.5 autoantibodies impair sodium current (INa) function, reducing conduction velocity and predisposing the myocardium to Brugada Syndrome (BrS)-like patterns and ventricular arrhythmias, as described in the scientific article titled "NaV1.5 autoantibodies in Brugada syndrome: pathogenetic implications" (Tarantino A. et al., European Heart Journal, 2024). Furthermore, from the above cited scientific article Geoffrey Lee et al., 2011 is know that anti-hERG (KCNH2) autoantibodies have been detected in patients with acquired Long QT Syndrome (LQTS), prolonging repolarization and increasing the risk of torsades de pointes.
- autoimmune inflammation and fibrosis: A chronic inflammation caused by persistent autoantibody production can lead to fibrosis, ion channel remodelling, and conduction abnormalities. The inventors of the present invention have extensively demonstrated that epicardial ablation over the right ventricular outflow tract (RVOT) can effectively eliminate the Brugada ECG pattern and reduce arrhythmic events. This approach directly targets the arrhythmogenic substrate, offering a complementary strategy to autoantibody modulation (Brugada J. et al., 2015: Pappone C. et al., 2017; Pappone C. et al., 2025) that the epicardial inflammation in BrS has been strongly associated with fibrofatty replacement of the right ventricular outflow tract (RVOT), an area where autoantibody-induced damage could play a central role. Therefore, fibrofatty replacement of the RVOT in Brugada Syndrome contributes to electrical heterogeneity, promoting conduction delay and arrhythmogenesis. Epicardial ablation not only eliminates arrhythmogenic substrate but may also mitigate chronic inflammatory signaling that sustains autoantibody production against NaV1.5. Also, from the scientific article titled "Tissue-resident memory T cells in epicardial adipose tissue comprise transcriptionally distinct subsets that are modulated in atrial fibrillation" (Vyas V. et al., 2024) is known that in atrial fibrillation (AF), local inflammatory cytokines and autoimmune responses against gap junction proteins (e.g., connexin-43) can disrupt impulse propagation, leading to atrial substrate instability.

In particular, in the field of onco-cardiology the identification of NaV1.5 autoantibodies in patients with metastatic breast cancer occurred. Recent studies, as the scientific articles with the title "Cardiac Cross-Reactivity of NaV Autoantibodies in Metastatic Breast Cancer: A Possible Trigger for Sudden Cardiac Death" (Pappone C. et al., 2024), have demonstrated that cancer-induced autoantibodies originally directed against tumor-associated antigens can cross-react with cardiac ion channels due to molecular mimicry, resulting in unexpected arrhythmic complications.

The mechanism of cross-reactivity involves tumor antigens sharing sequence or structural similarities with NaV1.5, which triggers an adaptive immune response that inadvertently targets cardiac sodium channels. In breast cancer, for example, NaV1.5 is overexpressed in metastatic cells, potentially priming the immune system to generate cross-reactive antibodies that subsequently recognize and disrupt myocardial NaV1.5 function. This unexpected onco-immune connection highlights a previously unrecognized cause of Brugada-like syndromes in cancer patients and suggests a potential paraneoplastic basis for certain arrhythmias.

These findings have significant clinical implications, as immunotherapy agents commonly used in oncology (e.g., checkpoint inhibitors) may exacerbate cardiac autoimmunity, increasing the incidence of immune-mediated arrhythmias. This highlights the critical necessity for developing targeted immune modulation approaches that can suppress autoimmune activity in the heart without causing systemic immunosuppression.

In general, standard-of-care treatments for cardiac arrhythmias primarily able only to suppress symptoms rather than targeting the underlying autoimmune pathology are known. These approaches include:
- pharmacological interventions with agents as antiarrhythmic drugs, β-blockers, calcium channel blockers: these agents effective for managing electrophysiological abnormalities, but these agents do not address autoantibody activity, making them insufficient in cases of autoimmune-mediated arrhythmias.
- catheter-based ablation: US Patents US D966507 and US11039773 describe advanced technologies for cardiac ablation. US Patent US D966507 pertains to a high-density catheter tip designed to improve ablation efficacy. Patent US11039773, on the other hand, focuses on advanced ablation techniques for treating cardiac arrhythmias, but the radiofrequency and cryoablation permanently destroy myocardial tissue, which may eliminate arrhythmic foci but does not prevent the immune system from attacking new cardiac regions.
- systemic immunotherapy with immunosuppressants like corticosteroids, intravenous immunoglobulin (IVIG), rituximab, et all., these immunosuppressants can reduce autoantibody titers, but their broad systemic effects may increase infection risk, impair immune surveillance, and disrupt normal cardiac function.

It is evident that traditional treatments fail to address the autoimmune nature of arrhythmias. Therefore, there is a critical need for targeted immune modulation that can neutralize pathogenic autoantibodies at the site of arrhythmogenesis while preserving normal myocardial function.

Therefore, epicardial adipose tissue (=EAT) plays a significant role in atrial fibrillation (AF) pathophysiology (Iacobellis G. Nature Reviews 2022). The close anatomical relationship between EAT and the atrial myocardium allows for direct interaction, contributing to the progression and maintenance of AF through several mechanisms. Firstly, EAT secretes pro-inflammatory cytokines that induce atrial fibrosis, leading to structural remodelling and the formation of a substrate for re-entry circuits. Secondly, recent evidence (Vyas et al., 2024)) suggests that resident memory T cells (T_RM) within EAT actively modulate local immune responses, potentially enhancing atrial arrhythmogenicity. Thirdly, a spatial correlation has been observed between left atrial EAT and areas of atrial fibrosis, indicating a synergistic effect in sustaining AF (Chahine Y. et al., 2022). The targeted ablation of atrial EAT may therefore represent a novel therapeutic approach by reducing both the inflammatory and structural substrates of AF.

In particular, autoimmune responses against connexin-43 and other gap junction proteins have been implicated in atrial fibrillation (AF) (Lazzerini PE, et al., 2025). These autoantibodies may contribute to electrical instability by disrupting gap junction integrity, reducing atrial conduction velocity, and increasing susceptibility to reentry. Epicardial adipose tissue, a known source of inflammatory cytokines, may further exacerbate this process, suggesting a potential role for targeted epicardial ablation in AF management, particularly in patients with an underlying inflammatory or autoimmune component.

Additionally, epicardial adipose tissue (EAT) has been implicated in the pathophysiology of heart failure with preserved ejection fraction (HFpEF). Its excessive accumulation is associated with increased myocardial stiffness and impaired diastolic function (Pugliese N.R. et al., 2021; van Woerden G. et al. ,2018). EAT has been increasingly recognized as a key contributor to diastolic dysfunction in HFpEF. Its pro-inflammatory profile and mechanical effects on ventricular compliance exacerbate myocardial stiffness. Targeted ablation of EAT may improve ventricular compliance and alleviate local inflammation, offering a novel therapeutic approach for HFpEF patients by directly modifying this pathological substrate, improving ventricular relaxation, and reducing local inflammation.

Epicardial adipose tissue infiltration has been implicated in arrhythmogenic cardiomyopathies, contributing to the development of arrhythmogenic substrates (Ernault A.C. et al., 2021). Targeted ablation of epicardial adipose tissue may reduce arrhythmic risk by modifying the pathological substrate responsible for electrical instability.

In the light of the above is desirable to provide a system able to simultaneously map, ablate, and/ or to deliver a therapy into a treatment area, and regulating the intensity of the electrical impulses, in order to minimize risks compared to traditional approaches, and allowing for targeted immune modulation.

### SUMMARY

It would be desirable to have a catheter-based system comprises a catheter able to map, ablate, and/or to deliver at least a therapeutic agent into at least a target area of a cardiac tissue, wherein the catheter includes a therapeutic ablator device disposed in a distal portion of the catheter, wherein the therapeutic ablator device comprises: a drug delivery portal, able to drug locally released; a plurality of railings arranged symmetrically and parallel to the drug delivery portal.

The invention further relates to a catheter-based system as detailed above for use in medical field in the treatment of arrhythmias and/or cardiac autoimmunity.

Additionally, the invention contemplates a catheter-based system as detailed above for use in medical field for targeting and/or ablation of epicardial adipose tissue.

It is a further object of the present invention the use of the catheter-based system as above detailed for targeting and/or ablation of epicardial adipose tissue, in the prevention and/or treatment of heart failure with preserved ejection fraction, arrhythmogenic cardiomyopathies with epicardial fat involvement.

Another object of the present invention relates to the use of the catheter-based system above detailed for the treatment of Brugada syndrome.

A further object of the present invention is the use of the catheter-based system above detailed for use in the prevention and/or treatment of structural heart disease such as arrhythmogenic right ventricular cardiomyopathy, hypertrophic cardiomyopathy, dilated cardiomyopathy with fibrotic remodeling, post-myocardial infarction scarring, calcific valvular stenosis with atrial remodeling, cardiac amyloidosis and surgically corrected congenital heart diseases (e.g., Tetralogy of Fallot) and onco-cardiology.

According to a further aspect, the subject-matter disclosed herein relates to a method of use of a catheter-based system, wherein the catheter is suitable to ablate and/or to deliver at least a therapeutic agent into at least a target area of a cardiac tissue of a patient to be treated, wherein the method comprising the steps of: inserting the catheter into the target area; propagating electrical impulses in the target area through a plurality of electrodes of a plurality of railings of the catheter, activating a circumferential balloon of the catheter, delimiting the target area; activating of a drug delivery portal of the catheter, and drug delivering into the target area through a plurality of vents of the drug delivery portal.

### BRIEF DESCRIPTION OF THE DRAWINGS

A more complete appreciation of the disclosed embodiments of the invention and many of the attendant advantages thereof will be readily obtained as the same becomes better understood by reference to the following detailed description when considered in connection with the accompanying drawings, wherein:
Fig. 1 shows a view of a first embodiment of an innovative catheter of an innovative catheter- based system.
Fig. 2 shows a plan view of a therapeutic ablator device of the innovative catheter of Fig. 1.
Fig. 3 shows a prospective view of a therapeutic ablator device of the innovative catheter of Fig. 1
Fig. 4 shows a prospective view of a drug treatment area into the distal portion of the innovative catheter of Fig. 1
Figs. 5, 5A and 5B shows a prospective view of a drug delivery portal and detailed features of a plurality vents of the innovative catheter of Fig. 1.
Fig. 6 shows a side elevation view thereof in a flexed position of the innovative catheter of Fig. 1.
Figs. 7, 7A and 7B show a plan view and lateral view of the preferred dimensional ranges and the cross-section view of the circumferential balloon preferred dimensional ranges of the catheter of the invention.
Figs.8-8D show a plurality side elevation view thereof in a a plurality of flexed positions of the innovative catheter of Fig. 1
Fig. 9 shows a flow chart of an embodiment of an innovative method of use of the catheter-based system of Fig. 1.

### DETAILED DESCRIPTION OF EMBODIMENTS

The catheter-based system of the invention includes a mapping and ablation catheter integrated with a drug delivery portal, enabling real-time substrate modification and localized therapy administration and/or immune modulation.

In particular, the innovative catheter-based system of the invention is able to map, ablate, and/or to deliver, targeted immunosuppressive therapy directly to the cardiac tissue addressing the root cause of autoimmune-mediated arrhythmias. The catheter-based system comprises an innovative catheter, the catheter comprises a plurality of flexible railings with mapping and ablation electrodes to provide real-time electrophysiological assessment, e to modify a cell membrane of the cardiac tissue to allow drug delivery, a drug delivery portal with calibrated vents to ensure uniform targeted immunosuppressive administration; and innovative circumferential balloon seals the treatment area, preventing immunosuppressive drugs from diffusing into adjacent tissues, thereby reducing collateral effects.

Unlike traditional ablation techniques, the catheter based system of the invention employs reversible electroporation and/or irreversible electroporation (pulse field ablation, PFA) regulating the intensity of the electrical impulses through an impulse generator of the catheter-based system, integrating precision immunotherapy with electrophysiological intervention, this catheter-based system provides a targeted, localized solution that directly neutralizes pathogenic autoantibodies at their site of action. By preserving myocardial integrity, preventing fibrosis, and reducing systemic adverse effects, this approach not only enhances therapeutic efficacy but also minimizes the risks associated with traditional ablative and immunosuppressive strategies, with implications in the treatment of arrhythmias and cardiac autoimmunity., in heart failure, structural heart disease, and onco-cardiology, where immune dysregulation plays a central role.

Reference now will be made in detail to embodiments of the disclosure, an example of which is illustrated in the drawings. Each example is provided by way of explanation of the disclosure, not limitation of the disclosure. In fact, it will be apparent to those skilled in the art that various modifications and variations can be made in the present disclosure without departing from the scope or spirit of the disclosure. In the following description, similar reference numerals are used for the illustration of figures of the embodiments to indicate elements performing the same or similar functions. Moreover, for clarity of illustration, some references may be not repeated in all the figures.

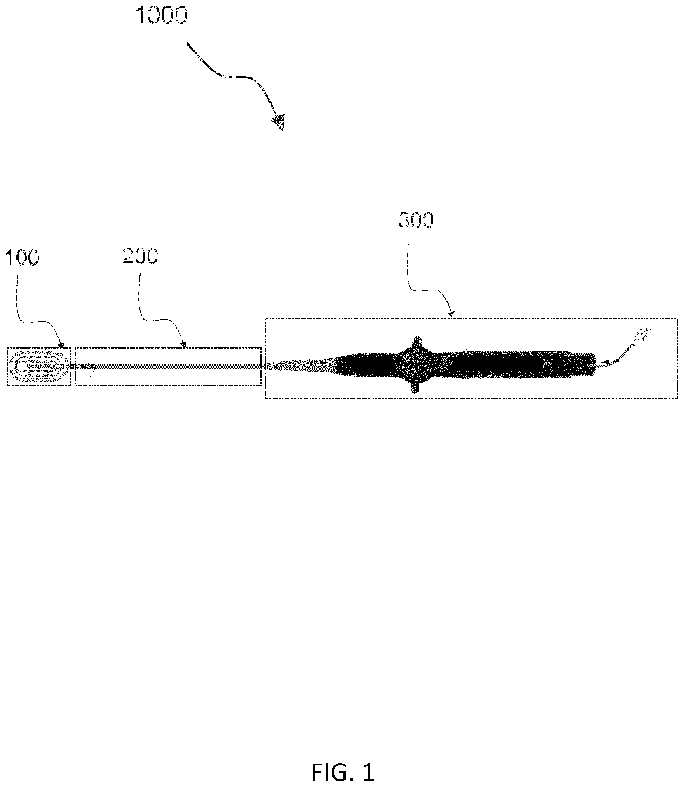

Fig. 1 schematically shows one embodiments of an innovative catheter-based system equipped with an innovative catheter. The innovative catheter is generally indicated with reference numeral 1000 in Fig. 1.

Considering Fig. 1 the catheter-based system comprises a catheter 1000, wherein the catheter 1000 is able to map, ablate, and simultaneously deliver at least a therapeutic agent into at least a target area of a cardiac tissue, preferably epicardium.

With non limiting reference to Fig. 1 the catheter 1000 is a flexible tubular shaft that includes a proximal portion 300 equipped with handle, a middle portion 200, and a distal portion that are connected in sequence,

According to preferred embodiment shown in Fig.1, the catheter 1000 includes a therapeutic ablator device 100 disposed in a distal portion of the catheter 1000.

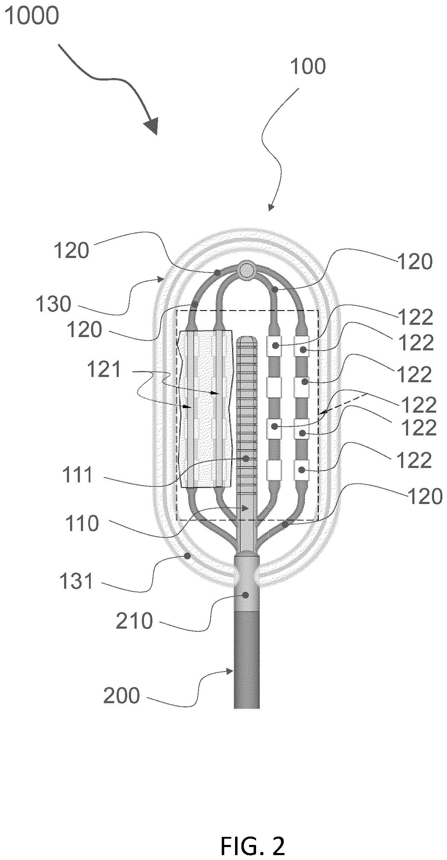

Fig. 2 shows that the therapeutic ablator device 100 comprises: a drug delivery portal 110, able to drug locally released; a plurality of railings 120 foreseeing two inner railings and two outer railings, and a circumferential balloon 130 around the therapeutic ablator device 100, so serve as a seal system when inflated to seal the treatment area and prevents drug diffusion into surrounding tissues thus reducing collateral tissue damage. In addition, ensuring that the delivered drugs remain localized within the treatment area 132.

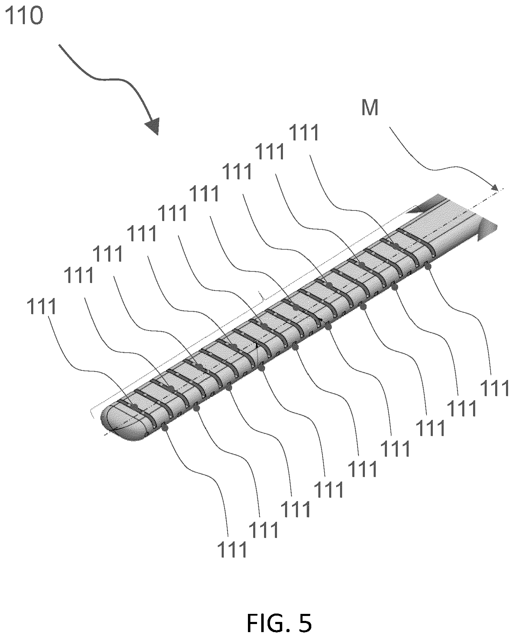

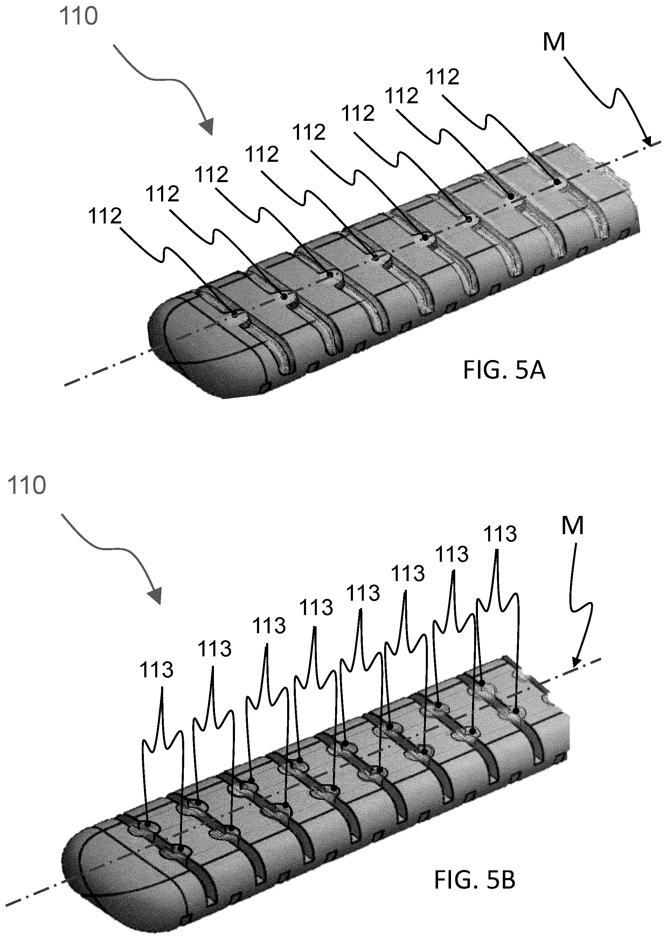

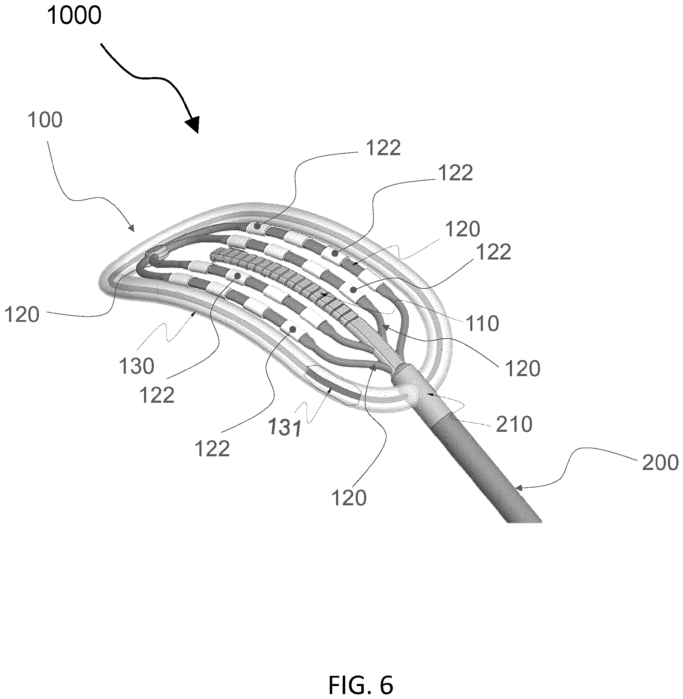

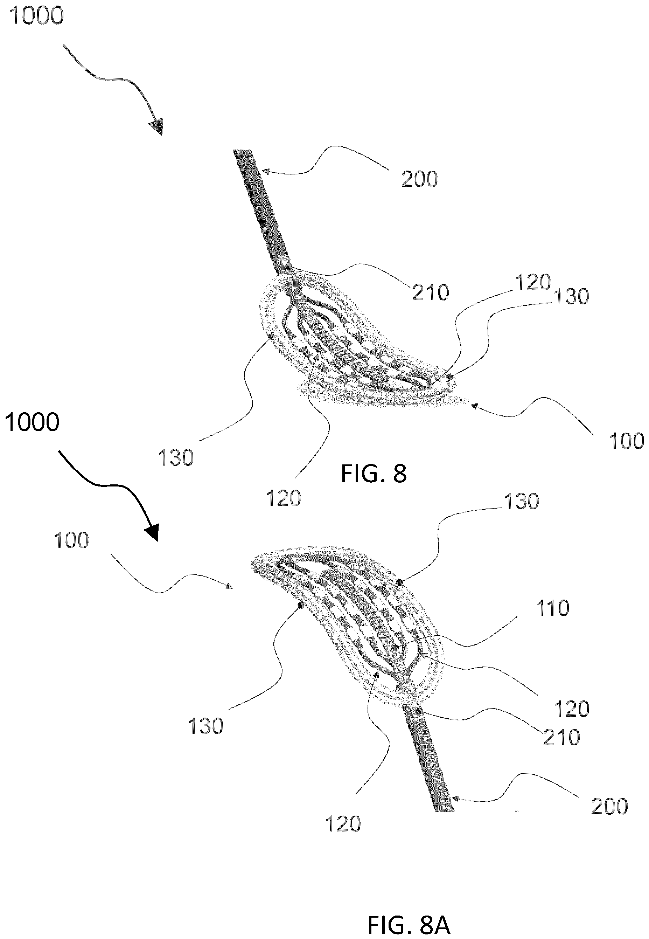

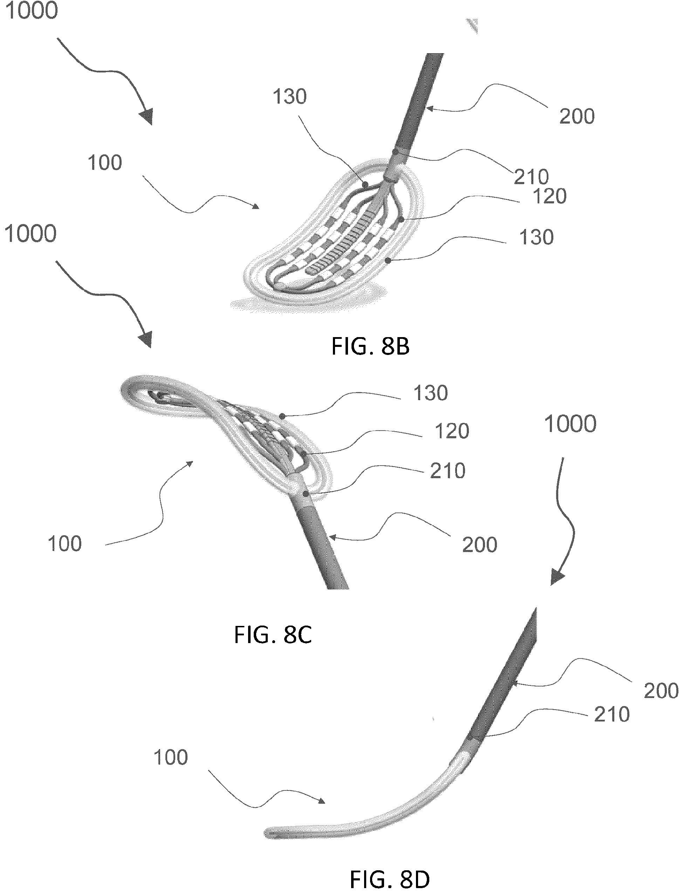

As shown in Figs. 5 and 6 the drug delivery portal 110 is flexible with an elliptical shape to move bi-directional vertical or radial motion, to conform to cardiac tissue, as shown in Figs.8-8D, preferably epicardium surface, and to move in -sync with cardiac motion and minimize horizontal or lateral curve.

It is to be noted that the drug delivery portal 110 is connected to one end of a middle portion 200 of catheter 1000 through a manifold 210 disposed in the middle portion 200, and shown in Fig. 5, it comprises a plurality of vents 111, preferably a plurality of calibrated vents.

Fig. 5 shows that the plurality of vents 111 is arranged with respect to the main axis M of the catheter 1000 at an angle α, where 90°-10° ≥ α ≥ 90°+10°, for bi-directional vertical or radial movement and for drug delivery in the target area.

Fig. 5 partially show without limitation that the plurality of vents 111 is equidistant and/or variable and with calibrated length openings and alternately positioned on opposite surfaces of the drug delivery portal 110, to provide bi-directional vertical or radial flexibility movement. In addition, the plurality of vents 111 may have a plurality of stand-offs 112 and/ or a plurality of holes 113 between each vents to keep each of the plurality of vents 111 continuously open and avoid delivery obstruction when the drug delivery portal is in curved position (as shown in Figs.5A and 5B).

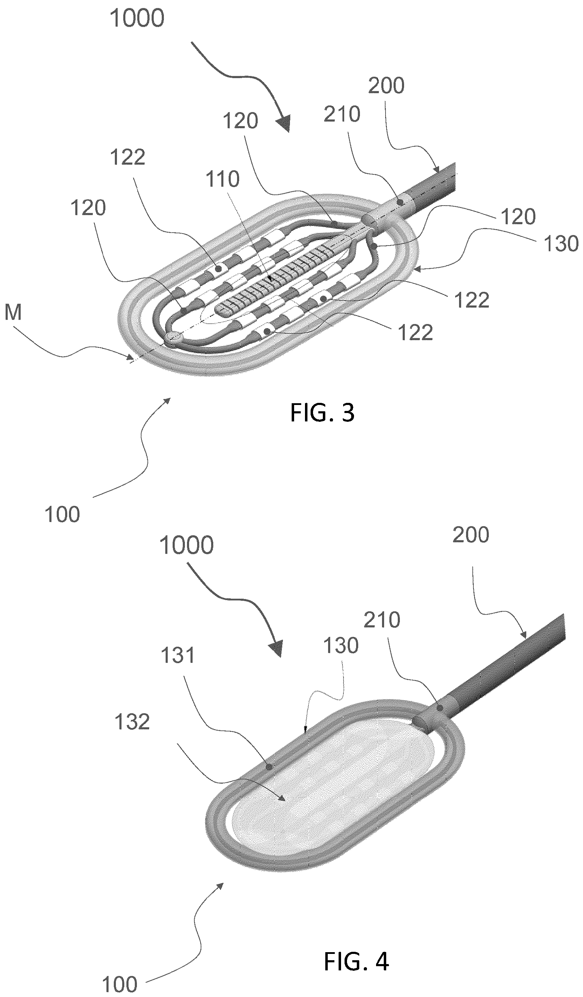

In particular, as shown in Figs 2 and 3.the plurality of railings 120 is flexible inner and outer railings arranged symmetrically and parallel to the drug delivery portal 110, preferably with an elliptical shape to conform to cardiac tissue, as shown in Figs.8-8D, in particular epicardium surface, to move in-sync with cardiac motion and minimize horizontal or lateral curve.

Furthermore, as shown in Fig. 2, the plurality of railings 120 comprises at least an internal structure support 121, to facilitate the housing of a plurality of electrodes 122, preferably the plurality of electrodes 122 is mapping and ablation electrodes. Advantageously, any combination of the plurality of electrodes is permitted. It is also possible that the plurality of electrodes can be used either for mapping or ablation, or both.

The internal structure support 121 is preferably a rectangular filar or a plurality of round filars or a combination of shapes of high memory material such as for example Nickel Titanium (NiTi). In addition, the internal structure support 121 is made with a material insulated with insulation with high dielectric properties such as polyimide or equivalent.

As shown in Fig. 6 the circumferential balloon 130 is equipped internally along the entire perimeter, with a balloon support structure 131, and a perimeter coating 133, wherein the circumferential balloon 130 preferable with an elliptical cross-section, allowing bi-directional vertical or radial motion and conforms to epicardium surface, as shown in Figs.8-8D, preventing the spread of the drug outside the treated area 132.

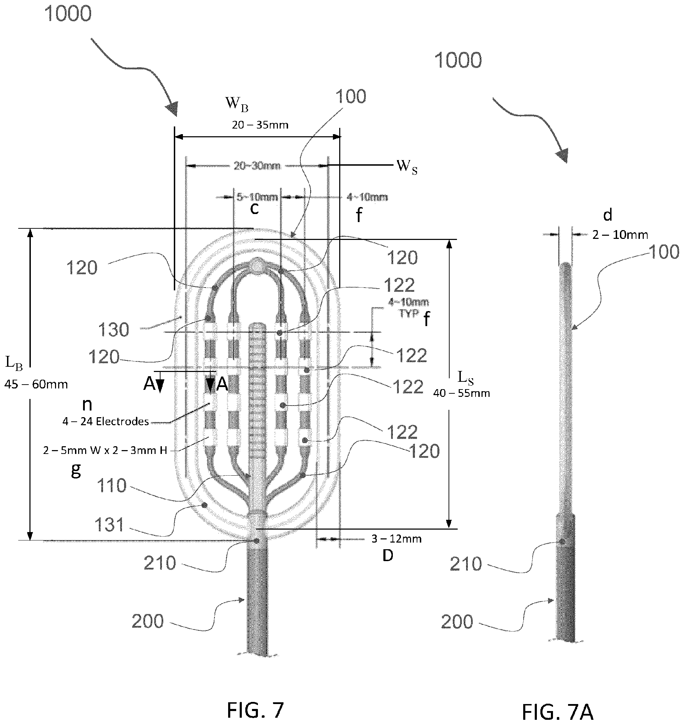

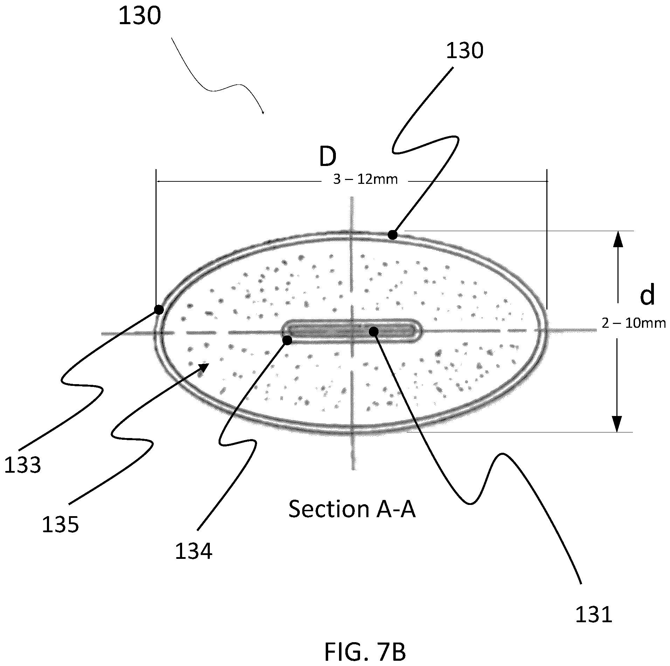

The balloon support structure 131 is preferably equipped with a rectangular filar, or plurality of round filars, or a combination of shapes made of high memory material such as Nickel Titanium (NiTi) or equivalent, to alleviate balloon folding and stretching and for fluorine recognition. As shown in Fig.7B the internal support structure is insulated preferably with a polymeric material 134 like fluoropolymer, thermoplastic polymer or equivalent. Therefore, the area 135 identified between the internal support structure 131 and the coating 133 is inflated through an inflating medium such as air, saline, or others

Advantageously, a minor diameter of the elliptical cross-section balloon is equal to or greater than a pericardial space or cavity. According to a preferred embodiment of the present invention, as shown in Fig. 7 and 7A, the width of the balloon internal support structure 131 assumes an overall numerical value of 20mm ≤ W_{S} ≤ 30mm and an overall length of 40mm ≤ L_{S} ≤ 55mm. The overall width of the circumferential balloon 130 assumes a numerical value of 20mm ≤ W_{B} ≤ 35mm and overall length of 45mm ≤ L_{B} ≤ 60mm. In another preferred embodiment the minor diameter of the elliptical cross-section balloon 130 assumes a numerical value of 2mm ≤ d ≤ 10mm and major diameter of 3mm ≤ D ≤ 12mm as shown in Fig. 7B. In Fig. 7B the cross-sectional shape of the circumferential balloon 130 can also be considered such as rectangular, square, circular or equivalent with diameter or height of cross-section shape equal to or greater than the pericardial space or cavity.

As shown in Fig. 1, the catheter 1000 includes a proximal portion 300, the middle portion 200, and the distal portion with the therapeutic ablator device 100 that are connected in sequence, wherein the therapeutic ablator device 100 is connected to the middle portion 200 through the manifold 210.

Advantageously, the manifold 210 is preferably a polymer manifold, it is used to channel the drug therapy to the treatment area 131 discrete from delivery channel for fluid or air to inflate the balloon. In addition, the distal portions of the plurality of flexible railings 120, one end of the drug delivery portal 110 and the ends of the circumferential balloon 130 are arranged within the manifold 210.

Furthermore, the catheter-based system is equipped with an impulse generator not shown in the figures, able of modulating the intensity and frequency of impulses emitted by the electrodes 122 of the plurality of railings 120, by switching from emitting short pulses of high-voltage energy when the catheter is an electroporation catheter, to pulses of low-voltage energy and the reverse, wherein the catheter is a pulsed -field ablation catheter.

Fig. 7 illustrates the preferred dimensional ranges of the catheter distal drug therapy system of the catheter of the invention. According to a preferred embodiment of the present invention, as shown in Fig. 7 and 7A, the distance between the electrodes located on the two side of the inner railing facing the drug portal system assumes a numerical value c with5mm≤c≤10 mm and the distance between the electrodes located on the same (inner or outer) railing assumes a numerical value f with 4mm≤f≤10 mm. According to another preferred embodiment of the present invention the total number of mapping and ablation electrodes assumes a numerical value n with 4≤n≤24. According to a particular preferred embodiment of the invention, as shown in Fig. 7 and 7A, the electrodes are shaped longitudinal semi-flat to allow bi-directional vertical or radial motion, to conform to epicardium surface, to move in-sync with cardiac motion and minimize horizontal or lateral curve. The width of the electrode 122 assumes a numerical value g with 2mm ≤ g ≤ 5mm and height of 2mm ≤ g ≤ 3mm. In Fig. 7, the shape of the electrode 122 can also be considered such as circular or equivalent with size numerical value g with 4F (French) 1.76mm ≤ g ≤ 8F (French) 2.7mm.

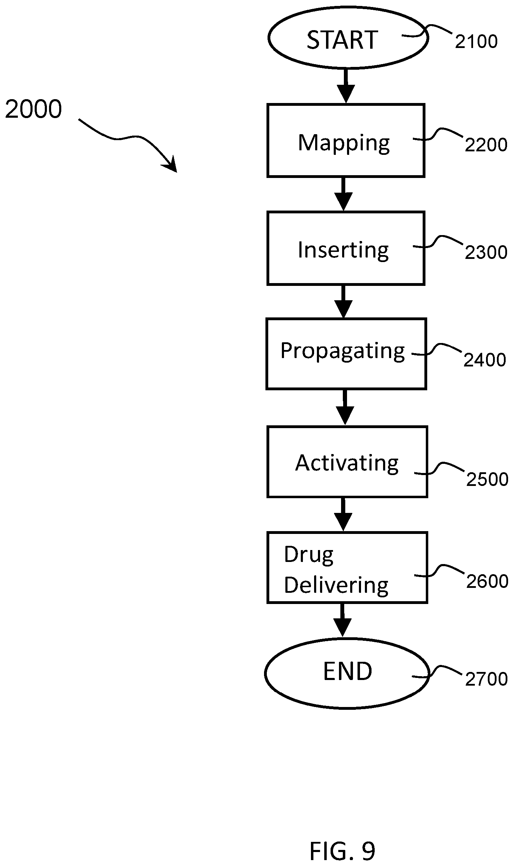

Fig. 9 illustrates a flow chart 2000 of an innovative method of use of a catheter-based system, wherein the catheter is able to ablate, and to deliver at least a therapeutic agent into at least a target area of a cardiac tissue of a patient to be treated, The flow chart has a start block 2100 and an end block 2700; the steps corresponding to block 2200 to block 2600 are typically repeated several times. Advantageously, the method (2000) comprising the steps of:
- inserting 2300 the catheter into the target area;
- propagating 2400 electrical impulses in the target area through a plurality of electrodes of a plurality of railings of the catheter,
- activating 2500 a circumferential balloon of the catheter, delimiting the target area.
- activating of a drug delivery portal of the catheter, and drug delivering 2600 into the target area through a plurality of vents of the drug delivery portal.

According to the embodiment shown in Fig. 9 the step of inserting 2300 is preceded by a step of mapping 2200 able to identify the target area, and in particular the step propagating 2400 electrical impulses in the target area is able to create at least a temporary pore in a cell membrane of at least a cell of the cardiac tissue in the target area, by emitting pulses of low-voltage energy through a plurality of electrodes.

As explained above, the step propagating 2400 electrical impulses in the target area creating a irreversible openings in a cell membrane of at least a cell of the cardiac tissue in the target area, by emitting short pulses of high-voltage energy through a plurality of electrodes, using a reversible electroporation-based catheter.

In addition, the step of activating (2500) the circumferential balloon occurs by inflating the balloon with air through the manifold connecting a delivery distal portion with a middle portion of the catheter, using the irreversible electroporation-based catheter.

Advantageously, the catheter-based system is able to modulate the intensity of electroporation, allowing for both irreversible (PFA) and/or reversible electroporation. Irreversible electroporation destroys cell membranes at high intensity, while reversible electroporation temporarily opens membranes to allow the entry of drugs, RNA, or DNA. This innovative approach could replace techniques like cryoablation and radiofrequency ablation.

Advantageously, the catheter-based system of the invention is particularly beneficial for the treatment of arrhythmias and/or cardiac autoimmunity. Beyond treating arrhythmias, this system can also be used for heart failure, structural heart disease, and onco-cardiology, establishing a new standard for targeted immune modulation in cardiac electrophysiology.

Epicardial adipose tissue (EAT) secretes pro-inflammatory cytokines that contribute to chronic low-grade inflammation, adversely affecting cardiovascular health (Patel K.H.K. et al., 2022).

Selective ablation of EAT may mitigate this inflammatory milieu, presenting a potential therapeutic strategy for conditions associated with metabolic inflammation.

Advantageously, epicardial adipose tissue (EAT) has been increasingly recognized as a key contributor to diastolic dysfunction in heart failure with preserved ejection fraction (HFpEF). Its pro-inflammatory profile and mechanical effects on ventricular compliance exacerbate myocardial stiffness. Targeted ablation of EAT may provide a novel therapeutic approach by directly modifying this pathological substrate, improving ventricular relaxation and reducing local inflammation

The innovative catheter-based system of the invention may be advantageously useful for targeting and/or ablation of epicardial adipose tissue, a type of adipose tissue near the heart that modulates the local immune system. As above indicated epicardial adipose tissue is metabolically active and secretes inflammatory substances that can negatively affect the heart, contributing to diastolic dysfunction and arrhythmias. Chronic inflammation of epicardial adipose tissue is associated with conditions like obesity and hypertension. Recent studies have shown a correlation between increased epicardial adipose tissue and diastolic dysfunction, as well as a link to atrial fibrillation (AF). Excess epicardial adipose tissue can promote fibrosis of atrial tissue and alter the heart's electrical conduction, increasing the risk of AF. Additionally, epicardial adipose tissue can infiltrate the atrial wall and influence the autonomic nervous system, facilitating the onset of arrhythmias.

The innovative catheter system expands treatment options for various immune-mediated arrhythmias, including but not limited to: Brugada Syndrome (BrS) involves preventing autoantibody-mediated NaV1.5 dysfunction and epicardial fibrosis; Long QT Syndrome (LQTS) addresses autoantibodies that prolong repolarization by targeting hERG (KCNH2) and CaV1.2 (CACNA1C) channels; Atrial Fibrillation (AF) modulates autoimmune inflammation in the epicardium; Early Repolarization Syndrome (ERS) prevents autoimmune modulation of Ito (transient outward potassium current); Cancer-Associated Arrhythmias neutralize cross-reactive NaV1.5 autoantibodies that arise from malignancies such as breast and colon cancer, mitigating arrhythmogenic effects without systemic immunosuppression; Post-Myocarditis Arrhythmias target persistent inflammatory-autoimmune responses that lead to chronic ventricular and atrial arrhythmias following viral myocarditis; Lethal Autoimmune Channelopathies address emerging arrhythmogenic syndromes linked to autoantibody-induced ion channel dysfunction, offering a novel immunomodulatory strategy for previously intractable conditions. According to other embodiments not shown in the figures:
- a catheter is introduced into the pericardial space and guided to locations where epicardial adipose tissue (EAT) accumulates. Said catheter is powered by an ultrasonic wave generator, operating in a frequency range of 2-6 MHz, delivering energy that selectively damages the epicardial fat, through clinically validated technologies used for non-invasive ultrasound lipolysis of subcutaneous adipose tissue.
- a catheter is introduced into the pericardial space and guided to areas of epicardial fat accumulation. Said catheter employs irreversible pulse field electroporation (IRE) energy with the following characteristics: voltage between 1,100-2,500 V, electric field strength >1,000 V/cm, pulse width ranging from 40-120 µs, pulse bursts of 50-100 pulses, and a monophasic or biphasic square waveform.
- a catheter is introduced into the pericardial space and directed toward epicardial fat tissue. Said catheter delivers localized cryotherapy, maintaining the target temperature between -65°C and -95°C, with a freezing duration of 3-5 minutes per cycle and employing 1-3 cycles. Cooling typically utilizes N2O or Argon expansion, achieving a cooling rate exceeding 30°C in the initial 20 seconds. Clinical and experimental evidence supporting cardiac cryoablation is robust.
- a catheter guidance within the pericardial space toward epicardial fat tissue relies on impedance mapping, identifying areas characterized by low electrical conductivity.

### REFERENCES

- Geoffrey Lee et al. Relationship among complex signals, short cycle length activity, and dominant frequency in patients with long-lasting persistent AF: a high-density epicardial mapping study in humans. Heart Rhythm, 2011; 8(11), 1714-1719.
- Monika Gawalko et al. Cardiac Arrhythmias in Autoimmune Diseases. Circ J. 2020; 84(5), 685-694.
- Lazzerini PE, et al. Autoimmune cardiac channelopathies and heart rhythm disorders: a contemporary review. Heart Rhythm, 2025.
- Tarantino A. et al. NaV1.5 autoantibodies in Brugada syndrome: pathogenetic implications. European Heart Journal, 2024; 45(40):4336-4348.
- Brugada J. et al. Brugada Syndrome Phenotype Elimination by Epicardial Substrate Ablation. Circ Arrhythm Electrophysiol. 2015; 8(6): 1373-81.
- Pappone C. et al. Electrical Substrate Elimination in 135 Consecutive Patients With Brugada Syndrome. Circ Arrhythm Electrophysiol 2017; 10(5).
- Pappone C. et al. Ablation of Epicardial Substrate in Brugada Syndrome Patients. J Am Coll Cardiol. 2025.
- Vyas V. et al. Tissue-resident memory T cells in epicardial adipose tissue comprise transcriptionally distinct subsets that are modulated in atrial fibrillation. Nat Cardiovasc Res, 2024; 3:1067-1082.
- Pappone C. et al. Cardiac Cross-Reactivity of NaV Autoantibodies in Metastatic Breast Cancer: A Possible Trigger for Sudden Cardiac Death. medRxiv, 2024.
- Iacobellis G. Nature Reviews Cardiology, 2022; Volume 19, pages 593-606.
- Chahine Y. et al., Epicardial adipose tissue is associated with left atrial volume and fibrosis in patients with atrial fibrillation. Front Cardiovasc Med. 2022; 9.
- Pugliese N.R. et al. Impact of epicardial adipose tissue on cardiovascular haemodynamics, metabolic profile, and prognosis in heart failure. Eur J Heart Fail. 2021; 23(11):1858-1871.
- van Woerden G. et al. Epicardial fat in heart failure patients with mid-range and preserved ejection fraction. Eur J Heart Fail. 2018; 20(11):1559-1566.
- Ernault A.C. et al., Modulation of cardiac arrhythmogenesis by epicardial adipose tissue: JACC state-of-the-art review. J Am Coll Cardiol. 2021; 78(17):1730-1745.
- Patel K.H.K. et al. Epicardial adipose tissue as a mediator of cardiac arrhythmias. Am J Physiol Heart Circ Physiol. 2022; 322(2):H129-H144.
- Vismaya S. Bachu1, et al. High-Intensity Focused Ultrasound: A Review of Mechanisms and Clinical Applications, Ann Biomed Eng. 2021 September ; 49(9): 1975-1991. doi:10.1007/s10439-021-02833-9.
- Jason G. Andrade, Cryoablation for atrial fibrillation, 2020;1:44-58

## Claims

1. A catheter-based system comprises a catheter (1000) able to map, ablate, and/or to deliver at least a therapeutic agent into at least a target area of a cardiac tissue,
wherein the catheter (1000) includes a therapeutic ablator device (100) disposed in a distal portion of the catheter (1000)
wherein the therapeutic ablator device (100) comprises:
- a drug delivery portal (110), able to drug locally released;
- a plurality of railings (120) arranged symmetrically and parallel to the drug delivery portal (110).

2. The catheter-based system of claim 1, wherein the therapeutic ablator device (100) comprises further a circumferential balloon (130) around the therapeutic ablator device (100).

3. The catheter-based system of claim 1, wherein the catheter (1000) is an electroporation catheter.

4. The catheter-based system of claim 1, wherein the catheter (1000) is a pulsed -field ablation catheter.

5. The catheter-based system of claim 2, wherein the circumferential balloon (130) is equipped internally along the entire perimeter, with a balloon support structure (131), wherein the circumferential balloon (130) prevents the spread of the drug outside a treated area (132).

6. The catheter-based system of claim 1, wherein the drug delivery portal (110) connected to one end of a middle portion (200) of catheter (1000) through a manifold (210) disposed in the middle portion (200), is a flexible drug delivery portal.

7. The catheter-based system of claim 1, wherein the drug delivery portal (110) comprises a plurality of vents (111), arranged with respect to the main axis (M) of the catheter (1000) at an angle α, where 90°-10° ≥ α ≥ 90°+10°.

8. The catheter-based system of claim 7, wherein the plurality of vents (111) is equidistant and/or variable with calibrated length openings and alternately positioned on opposite surfaces of the drug delivery portal (110).

9. The catheter-based system of claim 8, wherein the plurality of vents (111) have a plurality of stand-offs (112) and/ or a plurality of holes (113) between each of the plurality of vents (111).

10. The catheter-based system of claim 1, wherein the plurality of railings (120) is flexible railings, and comprises at least an internal structure support (121), to facilitate the housing of a plurality of electrodes (122).

11. The catheter-based system of claim 9, wherein the plurality of electrodes (122) is mapping and ablation electrodes.

12. The catheter-based system of claim 1, wherein the catheter (1000) includes a proximal portion (300), the middle portion (200), and the distal portion with the therapeutic ablator device (100) that are connected in sequence, wherein the therapeutic ablator device (100) is connected to the middle portion (200) through the manifold (210).

13. The catheter-based system of any preceding claims, wherein the catheter-based system is equipped with an impulse generator able of modulating the intensity and frequency of impulses emitted by the electrodes (122) of the plurality of railings (120), by switching from emitting short pulses of high-voltage energy to pulses of low-voltage energy and the reverse.

14. The catheter-based system of any preceding claims, suitable for deliver at least a therapeutic agent into at least a target area of a cardiac tissue of a patient suffering from arrhythmias and/or cardiac autoimmunity.

15. The catheter-based system of any of preceding claims 1-13, suitable for targeting and/or ablation of epicardial adipose tissue in a patient suffering from heart failure with preserved ejection fraction, Brugada syndrome, atrial fibrillation or arrhythmogenic cardiomyopathies with epicardial fat involvement.

16. The catheter-based system according to anyone of claims 1 to 13, suitable for ablation in a patient suffering from structural heart disease or in onco-cardiology.

17. A method (2000) of use of a catheter-based system according to anyone of claims 1 to 13, wherein the catheter is able to ablate and/or to deliver at least a therapeutic agent into at least a target area of a cardiac tissue of a patient to be treated, wherein the method (2000) comprising the steps of:
- inserting (2300) the catheter into the target area;
- propagating (2400) electrical impulses in the target area through a plurality of electrodes of a plurality of railings of the catheter,
- activating (2500) a circumferential balloon of the catheter, delimiting the target area.
- activating of a drug delivery portal of the catheter, and drug delivering (2600) into the target area through a plurality of vents of the drug delivery portal.

18. The method of claim 17, wherein the step of inserting (2300) is preceded by a step of mapping (2200) able to identify the target area.

19. The method of claim 17, wherein the step propagating (2400) electrical impulses in the target area is able to create at least a temporary pore in a cell membrane of at least a cell of the cardiac tissue in the target area, by emitting pulses of low-voltage energy through a plurality of electrodes.

20. The method of claim 17, wherein the step propagating (2400) electrical impulses in the target area creating an irreversible openings in a cell membrane of at least a cell of the cardiac tissue in the target area, by emitting short pulses of high-voltage energy through a plurality of electrodes.

21. The method of claim 17, wherein the step of activating (2500) the circumferential balloon occurs by inflating the balloon with air through the manifold connecting a delivery distal portion with a middle portion of the catheter.

22. The method of claim 17, wherein the target area is an epicardial adipose tissue, wherein the catheter employs delivers by an ultrasonic wave generator and/or an irreversible pulse field electroporation (IRE) and/or a localized cryotherapy, and/or an impedance mapping.
